# EUROPEAN PATENT SPECIFICATION

(11) **EP 3 088 009 B1**
(45) Date of publication and mention of the grant of the patent: **18.07.2018**
(21) Application number: 16167531.9
(22) Date of filing: 28.04.2016
(51) Int. Cl.: A61L 15/26, A61L 15/46, A61L 15/58

(54) **ANTIMICROBIAL ADHESIVE DRESSINGS**
ANTIMIKROBIELLE HEFTPFLASTER
PANSEMENTS ADHÉSIFS ANTIMICROBIENS

(30) Priority: 28.04.2015 US 201562153868 P
(43) Date of publication of application: 02.11.2016
(73) Proprietor: DiCosmo, Frank, Richmond Hill, ON L4C 7S4 (CA)
(72) Inventor: DiCosmo, Frank, Richmond Hill, ON L4C 7S4 (CA)
(74) Representative: Gassner, Birgitta

(56) References cited:
- EP-A1- 2 995 287
- WO-A2-2011/156910
- US-A- 4 643 181
- SIMON FINNEGAN ET AL: "EDTA: An Antimicrobial and Antibiofilm Agent for Use in Wound Care", ADVANCES IN WOUND CARE, vol. 4, no. 7, 1 July 2015 (2015-07-01), pages 415-421, XP55287554, ISSN: 2162-1918, DOI: 10.1089/wound.2014.0577
- T. KOBURGER ET AL: "Standardized comparison of antiseptic efficacy of triclosan, PVP-iodine, octenidine dihydrochloride, polyhexanide and chlorhexidine digluconate", JOURNAL OF ANTIMICROBIAL CHEMOTHERAPY., vol. 65, no. 8, 15 June 2010 (2010-06-15), pages 1712-1719, XP55287917, GB ISSN: 0305-7453, DOI: 10.1093/jac/dkq212

## Description

### Field of the Invention

The invention relates to adhesive dressings. More specifically the invention relates to transparent silicone adhesive dressings that are antimicrobial and may comprise a backing layer. The antimicrobial sheet dressing and pressure sensitive adhesive dressings are for use on wound areas and surgical incisions and as a protective dressing to cover for example indwelling therapeutic devices.

### Background of the Invention

The desirability of incorporating antimicrobial or antibacterial agents into different types of wound dressings is an important aspect of modern clinical medicine. Although numerous antimicrobials are available, most of these antimicrobial agents are either not suitable for long-term contact with human skin, or may have undesirable effects when used on open wounds, or there is difficulty in incorporating them into an adhesive material used as a contact layer of a wound dressing that is applied to the skin. For example, chlorhexidine is not desirable to use with open wounds as it can cause skin irritation when used in high doses. Silver antimicrobial agents have the downside of reported bacterial resistance making the use of silver-containing dressings not an attractive choice in clinical settings (Butchers, M. PHMB: an effective antimicrobial in wound bioburden management. British Journal of Nursing, 2012 (tissue viability Supplement), Vol 21, No 12, S16-S21.)

WO 2011/156910 discloses an antimicrobial self-adhesive gel sheet cured from a liquid containing silicone, the sheet having dispersed therein particulates of a chlorhexidine compound that is not soluble in the liquid; and at least one other antimicrobial which is one or both of chlorhexidine digluconate and a photo-stabilized silver agent. The use of PHMB in wound management is relatively new. PHMB is an antimicrobial with antiseptic efficiency for which negative effects have not been reported (Gilliver, S. PHMB: A well-tolerated antiseptic with no reported toxic effects: Koburger *et al*. [Koburger, T.; Hübner, N.-O.; Braun, M.; Siebert, J.; Kramer, A. Standardized comparison of antiseptic efficacy of triclosan, PVP-iodine, octenidine dihydrochloride, polyhexanide and chlorhexidine digluconate. J. Antimicrob. Chemother. 2010, 65, 1712-1719).

Several different types of medical dressings are used in wound care. Pressure sensitive adhesives (PSAs) are dressings used in wound care that have the advantage of not resisting separation when the adhesive is peeled off skin or tissue. Medical dressings may be fabricated to contain various types of therapeutics including antimicrobials as disclosed for example in: US 4,643,181; US 5,976,117; US 5,817,325; US 6,572,878; US 6,017,561; US 7,799,765; US 7,750,201; US 7,704,523; US 7,977,403; US 8,147,857; US 8,672,906; and US 2012/0294920.

Commercially available pressure sensitive dressing materials include OpSite (Smith & Nephew,IIc), Tegaderm (3M, USA), and Medifix® (Scapa, USA) with acrylic-based pressure sensitive adhesives or similar material. These dressings have relatively strong adhesives that stick exceedingly well to human skin and upon removal can cause additional trauma to wound sites and damaged skin, as well as causing pain upon removal by pulling on sensitive skin and or hair.

There still remains a need in clinical medicine to provide a pressure sensitive wound dressing that is adhesive and provides a combination of properties that may be useful in the treatment of a variety of wounds resultant from several different types of clinical indications. It is further desired to provide a pressure sensitive adhesive dressing that incorporates hydrophilic materials in a manner more useful for wound management.

### Summary of the Invention

According to one aspect of the present invention, there is provided a silicone adhesive dressing comprising a transparent and self-adhesive silicone material made from a liquid containing silicone, comprising therein a PHMB compound and EDTA that is not soluble in the liquid silicone material.

PHMB is a desired antimicrobial for use in the present invention. Furthermore, a chelating agent such as ethylenediamine tetra-acetic acid (EDTA) may be added as the desired metal chelator. EDTA is added as a preservative and is included in combination with the PHMB. EDTA as well as acting as a preservative is a well-known inhibitor of matrix metaloproteases (MMPs) in that it chelates zinc ion from the active site of MMPs rendering them inactive.

A wound dressing of the present invention may further optionally include another chelating agent. Any suitable chelating agent may be used. Chelating agents such as ethylenediaminetetraacetic acid (EDTA), and variations of EDTA such as, disodium EDTA or tetrasodium EDTA, and combinations thereof and the like, are contemplated. Chelating agents are known to increase antiseptic effects thereby rendering the wound dressing more effective in preventing infection. One of ordinary skill in the art will readily be able to select an appropriate amount of the selected antimicrobial, and EDTA/chelator for inclusion in adhesive layer.

The present invention is applicable for use to surgical incision site wound dressings and intravenous (i.v.) needle sites generally, or any wound covering where a transparent antiseptic silicone sheet may be used, such as an open wound in which negative pressure wound therapy would be applied, or a surgical drape. The presence of an antimicrobial in the adhesive portion of such dressings prevents possible migration of bacteria that may be present on the skin at or near the site of the wound, incision or catheter exit site, needle insertion site, into the wound and helps prevent infection of said wound.

The present invention may be used with any substrate generally used for making surgical dressings or i.v. dressings, or any wound covering where a transparent antiseptic silicone sheet may be used, such as an open wound in which negative pressure wound therapy would be applied, or a surgical drape. The substrate may be a thin transparent polymer material such as a film backing material, woven or knitted fabric, a nonwoven fabric or a plastic or polymeric material such as cotton, carboxymethylcellulose, biocellulose, collagen, and gelatin as is generally known in the art. A desirable material in the present invention is polyurethane backing with a high moisture-vapour transmission rate such as PS-1083 polyurethane film from Polymer Science, Inc., Monticello, IN, USA.

The antimicrobial employed in the present invention in aspects is polyhexamethylene biguanide hydrochloride, PHMB. This material is commercially available as COSMOCIL CQ from Lonza. The chelator is EDTA and is widely available.

Dressings of the invention have use to cover surgical incision wounds, i.v. puncture sites, open wounds in negative pressure wound therapy applications, preoperative surgical drapes, and the like, as well as in other wound dressing applications including burn dressings, chronic wounds, skin and tissue ulcers and the like.

In aspects of the invention, the dressings of the invention exhibit one or more of the following: tackiness, transparency, flexibility, anti-microbial effects for several days and flexibility. The dressings of the invention are repositionable as they remain adhesive with little to no tissue trauma.

According to an aspect of the invention is an antimicrobial silicone adhesive dressing, the dressing comprising:
- a silicone material comprising PHMB and EDTA, wherein said PHMB and EDTA are not substantially soluble in the silicone material,
- a backing layer; and
- an optional liner laminated to a tissue contact surface of the silicone material.

In aspects, a humectant such as glycerin is further incorporated into the silicone material to help preserve moisture.

The silicone material functions as a tissue contact layer, as it is adhesive and will adhere to a tissue or organ to promote healing, protection as a cover and to aid in the placement and/or protection of indwelling therapeutic devices.

According to a further aspect of the invention is antimicrobial silicone adhesive dressing that comprises a film backing material and a tissue contact layer thereon, wherein said tissue contact layer comprises silicone comprising particulates of PHMB (polyhexamethylene biguanide hydrochloride) insoluble in said layer, EDTA (ethylenediamine tetra-acetic acid) and optionally at least one other antimicrobial and optional humectant.

According to a further aspect of the invention is antimicrobial silicone adhesive dressing that comprises in order:
- a backing layer;
- a silicone layer thereon, comprising PHMB (polyhexamethylene biguanide hydrochloride), EDTA (ethylenediamine tetra-acetic acid) that are insoluble in said silicone, glycerin and optionally at least one other antimicrobial; and
- an optional liner laminated onto said silicone layer.

In aspects, the liner is a polycarbonate material.

According to a further aspect of the invention is antimicrobial silicone adhesive dressing that comprises a film backing material and a self-adhesive silicone layer thereon, wherein said tissue contact layer comprises particulates of PHMB (polyhexamethylene biguanide hydrochloride) insoluble in said layer, EDTA (ethylenediamine tetra-acetic acid) and optionally at least one other antimicrobial, and further wherein said self-adhesive silicone layer is in the form of a pad, foam, thin film or gel.

According to a further aspect of the invention is antimicrobial silicone adhesive dressing that comprises a film backing material (baking layer) and a self-adhesive silicone layer thereon, wherein said tissue contact layer is transparent, flexible and comprises particulates of PHMB (polyhexamethylene biguanide hydrochloride) insoluble in said layer, EDTA (ethylenediamine tetra-acetic acid), glycerin and optionally at least one other antimicrobial,
wherein said film backing material is permeable to air and moisture vapor and substantially impermeable to liquids, microorganisms and viruses.

According to another aspect of the invention is a method for making a silicone adhesive wound dressing, the method comprising:
- mixing a liquid containing silicone with a dispersion of PHMB and EDTA not soluble in said liquid;
- adding platinum as a catalyst to cure said mixture;
- forming said cured mixture as a thin film layer, pad, foam, or gel onto a film backing material and optionally laminating a liner to a surface of the cured layer, pad, foam or gel.

According to a further aspect of the present invention, there is provided an antimicrobial adhesive silicone dressing for use in a method for treating wound areas and surgical incisions or for preventing or minimizing infection of a wound or incision site or needle puncture, the method comprising contacting the wound, the surgical incision or the needle puncture with the antimicrobial adhesive silicone dressing. In aspects the silicone adhesive dressing further comprises a humectant such as glycerin.

According to an aspect of the invention is an adhesive dressing comprising a conformable backing layer having an antimicrobial adhesive silicone layer thereon, the adhesive layer comprising PHMB, EDTA and glycerin.

According to a further aspect of the invention is the use of a combination of PHMB and EDTA in the manufacture of a transparent antimicrobial silicone adhesive wound dressing, the dressing being transparent and flexible. The PHMB and EDTA remain as particulates (i.e. particles) within the silicone.

According to an aspect of the invention is a method for making an antimicrobial silicone adhesive dressing, comprising mixing together a liquid containing silicone with a dispersion of polyhexamethylene biguanide hydrochloride (PHMB) that is not soluble in said liquid; and allowing the mixture to cure. In aspects the curing is effected in the presence of a catalyst. In aspects, the catalyst is platinum. In aspects, about 0.1 to about 1%, or from about 0.3 to about 0.5% or about 0.3% of PHMB is present in the cured adhesive dressing. In aspects the PHMB dispersion is provided as a powder (Arch Chemicals USA). The method further comprises adding EDTA to said PHMB dispersion, wherein mixing yields a cured silicone material having about 0.1% EDTA. In aspects the method further comprises adding a pharmaceutical agent to said PHMB dispersion. In aspects the cured silicone material is provided as a sheet. In aspects the sheet is coated onto a backing material. In aspects the backing material is polyurethane. In further aspects a liner is laminated to the cured silicone material.

According to an aspect of the invention is a method for making an antimicrobial adhesive silicone for an adhesive dressing, the method comprising mixing together;
a liquid containing silicone with a silicone dispersion of polyhexamethylene biguanide hydrochloride (PHMB) and EDTA that are not soluble in said liquid and remain as particulates, and optional humectant;
and allowing the mixture to cure under suitable temperature and pressure.

In any aspects the cured antimicrobial adhesive silicone comprises about 0.1 to about 1%, or from about 0.3 to about 0.5% or about 0.3% of PHMB.

In any aspects the cured antimicrobial adhesive silicone comprises up to about 0.1% EDTA, or from about 0.01 to about 1%, or from about 0.1 to about 0.5% EDTA.

In any aspect the humectant is present in an amount of up to about 2% in said cured antimicrobial adhesive silicone.

In any aspects the humectant is glycerin.

In any aspects the curing is effected in the presence of a platinum catalyst.

Any aspects further comprise adding a pharmaceutical agent to said dispersion.

In any aspects of the method disclosed herein the mixture is coated onto a backing material prior to curing.

In any aspects the backing material is selected from the group consisting of polyurethane, polyester, vinyl, cellulose, oxycellulose, rayon, viscose and composite biomaterials.

In any aspects the backing material is polyurethane.

In any aspects a liner is laminated to said cured antimicrobial adhesive silicone on a tissue and/or skin contact surface. The invention relates to a transparent antimicrobial silicone wound dressing made by the methods disclosed herein, wherein said wound dressing is self-adhering, flexible, transparent, re-positionable and washable.

A further aspect of the invention is an adhesive antimicrobial dressing comprising:
a transparent and adhesive silicone sheet or film cured from a liquid containing silicone, the sheet comprising humectant and dispersed particulates of polyhexamethylene biguanide hydrochloride (PHMB) and EDTA that are not soluble in said liquid containing silicone;
a backing layer on one side of said sheet; and
an optional liner on a tissue and/or skin contact side .

In aspects the amount of PHMB in said sheet is up to about 5% by weight of said dressing.

In aspects the amount of EDTA in said sheet is about 0.1% EDTA, or from about 0.01 to about 1%, or from about 0.1% to about 0.5%

In aspects the humectant is glycerin provided in an amount of up to 2% v/v.

In aspects the dressing has a thickness of up to about 5mm.

In aspects the dressing is transparent, tacky, flexible, repositionable and washable.

In aspects the backing layer is made of a material selected from the group consisting of polyurethane, polyester, vinyl, cellulose, oxycellulose, rayon, viscose and composite biomaterials.

In an aspect of the invention is an antimicrobial adhesive silicone of the invention for use in a method for treating wound areas and surgical incisions or for preventing or minimizing infection of a wound or incision site or needle puncture, the method comprising contacting the wound, the surgical incision or the needle puncture with the antimicrobial adhesive silicone. In aspects of the method the invention further comprises applying said antimicrobial adhesive silicone to an indwelling therapeutic device as a protective dressing.

In any aspects the backing layer is vapour permeable.

In any aspects the backing layer comprises a hydrophilic polyurethane having a water uptake of 5 to 95% by weight of water.

In any aspects the liner is polycarbonate.

In any aspects the dressing provides microbiocidal activity of said PHMB for at least up to 7 days.

### Brief Description of the Drawing:

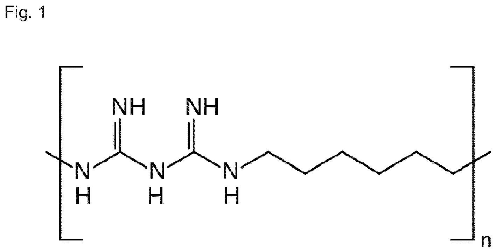

Figure 1 shows oligomers of PHMB.

### Detailed Description of the Invention

Herein described are novel wound dressings that are antimicrobial and adhesive. The dressings have a layer that adheres to skin and tissue. The dressing can be further fabricated to have a film backing material and further formulated to have a liner laminated to the adhesive surface.

The antimicrobial silicone adhesive dressings in general comprise a tissue contact layer that is a hydrophobic material. An antimicrobial is dispersed with the tissue contact layer in a manner that the antimicrobial is not soluble therein and instead forms particulate antimicrobial particles dispersed therein. To the antimicrobial is provided a chelator, humectant and optionally other antimicrobial agents. The dressings are silicone based, in aspects, a silicone gel material that can be provided in a variety of formats.

PHMB is utilized as the antimicrobial and is used in conjunction with EDTA in order to deter microbial ingress and growth on or with a wound dressing, the wound bed, the surface surgical wound excision site, or dermal and/or tissue exit-sites of a medical device such as a topical surgical dressing, negative pressure wound therapy dressing or contact layer, or ostomy wound contact devices or covering film and/or catheter exit site wounds such as those of venous catheters and the like.

Surprisingly, the silicone-based adhesive dressing of the present invention retains the desired adhesive and beneficial properties while admixed with a dispersion of insoluble PHMB and EDTA, it is known that EDTA is a metal chelating compound.

The present invention is the first to realize that a transparent tacky silicone gel can be combined with an antimicrobial in combination with a metal chelating agent and humectant to form a stable composition for the manufacture of a dressing for covering surgical incisions, i.v. site wounds, wounds requiring NPWT and other wounds. Still other antibiotics may be further combined with metal chelators and silicone compositions to manufacture medical devices such as surgical dressings, i.v. dressings, surgical drapes, NPWT dressings, and the like for treating wounds; for example, chlorhexidine in combination with EDTA may be combined with the silicone as described in this invention to provide a composition that may be manufactured into a medical devices noted above.

The invention provides a transparent silicone sheet dressing such as a wound dressing, that includes PHMB and EDTA that are insoluble in the silicone polymer used to form the adhesive dressing skin or tissue contact layer, resulting in insoluble particles dispersed throughout the resulting adhesive dressing. A PHMB and EDTA dispersion in the tacky silicone adhesive mixture allows for incorporation of antiseptic PHMB and EDTA combination into the adhesive wound dressing or skin covering thus providing a preservative function within the adhesive contact layer and antimicrobial action of the dressing when placed on human skin and tissue.

The antimicrobial silicone adhesive dressings of the invention provide gentle, atraumatic adhesiveness, and topical antiseptic activity for several days. The dressings are transparent and this allows for visibility of a wound site or other surgical incisional site, or i.v. needle puncture site, skin surface or wound without having to lift the dressing from the skin, wound or incision site. Its gentle adhesion and flexibility allow it to be easily lifted and removed cleanly from patients' without skin trauma or damage to sensitive and/or inflamed wounded tissue, no matter how long the silicone adhesive has been on the skin. As silicone gel does not lose adhesion when removed from the skin, it can be washed with water, air dried, and reapplied to the skin if required.

In other embodiments of the invention, the tissue contact layer is a hydrophobic silicone material formatted as a silicone adhesive, silicone gel, silicone thin film, silicone pad, or silicone foam.

Silicones are polymers consisting of alternate atoms of silicon and oxygen with organic groups attached to the silicon atoms. The degree of polymerisation determines the physical form of the silicone, which can vary from thin oils to relatively hard rubbers or soft sticky resins. Soft silicones are soft and tacky and adhere well to dry surfaces. A soft silicone wound dressing is coated with a soft silicone adhesive or a wound contact layer that can be removed without causing trauma to the wound or to the surrounding skin. The dressing may be easily lifted from the skin and re-applied to the dry skin with no appreciable reduction to the adhesive strength.

The invention as an antimicrobial silicone adhesive dressing is transparent and comprises: 1) an anti-microbial component comprising at least PHMB; 2) a hydrophobic silicone polymer; 3) at least EDTA in combination with PHMB, 4) optional humectant such as glycerin where the polymer comprises a medical grade silicone that can be platinum-cured to a tacky adhesive used in medical applications such as a wound dressing for surgical wounds, i.v. needle insertion sites, surgical drapes, negative pressure wound therapy (NPWT) adhesive coverings, NPWT tissue contact layers, surgical drapes and the like. It should be recognized by those experienced in the art that other suitable medical devices may be produced by the invention herein.

Suitable silicone adhesive material for use in the invention are platinum-catalyzed, fillerless, silicones elastomer adhesives such as Dow Corning BIO-PSA class of silicone adhesives and Dow Corning silicone Soft Skin Adhesives (SSAs) MG 7-9800, MG 7-9850 and MG 7-9900. In one aspect MG 7-9900 is a desirable silicone. Other examples of adhesive silicone gel compositions of clear, tacky silicones suitable to practice the invention herein include the two component MED-6345™ tacky silicone gel by Nusil Technology LLC, CA, USA, and Wacker SILPURAN® 2112 A/B, 2120 A/B and 2130 A/B, 2-part, addition-curing silicone compositions curing to soft, tacky silicone adhesives by Wacker Chemie GmbH, Munich, Germany. Components A and B of SILPURAN® 2110 A/B and 2120 A/B for example are mixed homogeneously in a ratio A : B = 1 : 1 and cure rapidly at temperatures over 100°C. The cured composition has good tackiness and adhesive properties. Increasing the proportion of component A results in a softer silicone gel and increased tackiness. Increasing the proportion of component B results in a harder gel with reduced tackiness. The platinum catalyst is in component A.

A solventless silicone adhesive for use in the present invention is a cross-linked silicone soft skin adhesive (SSA) elastomer technology. SSA materials are known as tacky gel or silicone gel. They differ from analogous silicone elastomers by the absence of reinforcing silica filler. As a result, they have the consistency of a gel but they are not truly polymeric gel because they are not based on an insoluble polymer network swollen with low molecular weight fluids. SSAs are cross-linked polydimethylsiloxanes with low amounts of free extractable molecules. Despite low consistency and some compressibility, SSAs show resilience and quick recovery under cyclic deformation. The pressure sensitive adhesive property of SSA is mainly based on the capacity of the surface to quickly wet the substrate and conform to its relief without excessive flow. Because the viscous component is minimal, the material does not flow, and only small dissipation of the energy occurs when deformation pressure is applied. The result is an immediate debonding, which happens at low peel or shear force. The advantage in skin adhesion is the atraumatic removal obtained with SSA: no skin stripping and no painful skin or hair pulling. Another advantage lies in the fact that SSAs have a low viscous component that limits their flow and consequently the readiness to absorb materials at the surface of the skin such as stratum corneum cells and lipids. The adhesive surface of SSAs remains relatively clean. It can be removed and re-adhered easily to the same location (Silicone Adhesives in Healthcare Applications, Thomas, X. Dow Corning Technical Brochure and references therein).

Cross-linking of SSAs is based on an addition reaction (hydrosilylation), between vinyl functional PDMS and hydrogen functional siloxanes (e.g. dimethyl, methylhydrogen siloxane copolymers, hydrogen dimethylsiloxy terminated PDMS) as shown in the Figure 6. The cure reaction is catalyzed by a platinum complex. It can occur at room temperature or be accelerated at elevated temperature (80°C to 145°C), without the formation of by-products.

In one embodiment of the present invention silicone adhesives (SSAs) are utilized as they are two-part, platinum-catalyzed, fillerless, high adhesion elastomeric silicone adhesives. They are clear and soft skin adhesives suitable for wound care applications, as they provide the following characteristics:
- Tack for quick bonding to various skin types, and wet skin;
- Suitable adhesiveness and cohesiveness;
- Gentle adhesion and atraumatic removal from fragile and compromised skin;
- No skin stripping or peeling for hair;
- Ability to reposition easily and re-apply easily and effectively;
- High degree of flexibility;
- Moisture and gas permeable;
- Compatibility with many therapeutic molecules; and
- Co-formulation with pharmaceutical excipients to adjust the kinetics of drug efflux.

Surprisingly is it now for the first time demonstrated that silicone elastomer solutions (such as for example but not limited to MG 7-9800, MG 7-9850 and MG 7-9900) may be combined with a polymeric biguanide, such as PHMB and at least EDTA to form a stable composition that can be cured by platinum-based catalysis. Prior to the present invention, it was not realized or demonstrated that PHMB and EDTA in combination could be effectively combined with a silicone liquid solution in a stable manner to form a stable solution with particulates that could be cured to form a transparent dressings for use in treating various wounds.

The backing onto which the silicone adhesive layer laid is preferably flexible film-like polymer material such as but not limited to polyurethane. The added soft silicone adhesive material will be the skin adhesive contact layer. The backing is preferably substantially permeable to air and moisture vapor. The backing is also preferably substantially impermeable to liquids and microorganisms and viruses. Examples of suitable materials for backing layer include, but are not limited to, polyurethanes, polyesters, and vinyls, cellulose, oxycellulose, rayon, viscose, collagen foams, pads, thin films, and composite biomaterials, and flexible thin films of any medically suitable and materials biocompatible with human skin and tissues.

Antimicrobial agents that may be used for the purposes of the invention may be any such agent which is suitable for use in the dressing. In addition, to the PHMB and EDTA, as a desired embodiment, at least one other antimicrobial agent may optionally be included in the dressing, with or without EDTA. Non-limiting examples include compounds of metals such as silver, copper, or zinc, iodine based compounds, polyhexamethylene biguanide (PHMB) and derivatives, chlorohexidine gluconate/acetate, and Octenidine and derivatives. Further alternative anti-microbial agents are possible. Suitable anti-microbial agents include, but are not limited to, a triclosan, a polymoxin, a tetracycline, an amino glycoside (e.g., gentamicin or Tobramycin™), a rifampicin, a bacitracin, an erythromycin, a neomycin, a chloramphenicol, a miconazole, a quinolone, a penicillin, a nonoxynol 9, a fusidic acid, a cephalosporin, a mupirocin, a metronidazole, a secropin, a protegrin, a bacteriolcin, a defensin, a nitrofurazone, a mafenide, a acyclovir, a vanocmycin, a clindamycin, a lincomycin, a sulfonamide, a norfloxacin, a pefloxacin, a nalidizic acid, an oxalic acid, an enoxacin acid, a ciprofloxacin, a biguanide, combinations thereof and the like. In certain embodiments the anti-microbial agent comprises polyhexamethylene biguanide (PHMB) and/or derivatives thereof.

In one particular embodiment of the invention, PHMB is utilized. Polyhexamethylene biguanide (PHMB, N-(3-aminopropyl)-imidodicarbonimidic diamide, or also known also known as polyhexanide poly(hexamethylenebiguanide hydrochloride), poly(iminocarbonimidoyliminocarbonimidoylimino-1,6-hexanediyl) hydrochloride, poly(iminoimidocarbonyl-iminoimidocarbonyl-iminohexamethylene) hydrochloride, poly(iminoimidocarbonyliminoimidocarbonyliminohexamethylene) hydrochloride, with the following trade names, Baquacil, Caswell No. 676, Cosmocil CQ, EPA Pesticide Chemical Code 111801, Polihexanido, Polihexanidum, Polyhexanide, PP 073 and UNII-322U039G has a proven efficacy against pathogens commonly found in wounds.

PHMB is an aqueous-based cationic compound used as a preservative and antimicrobial agent, it is active against microorganisms, and is compatible with a wide range of aqueous-based cosmetics and personal-care products. Preparations of PHMB (Figure 1, where n is an integer) are mixtures of polymeric biguanides with a molecular weight range of 400-8,000 representing polymers with 2-40 repeating subunits, with an average number 11 repeating subunits (Figure 1, n=11) (Cationic Antimicrobial Polymers and Their Assemblies A. M. Carmona-Ribeiro, L. Dias de Melo Carrasco, Int. J. Mol. Sci. 2013, 14, 9906-9946).

Due to dynamic equilibrium of polymerization/depolymerization in step-growth synthesis, commercial PHMB has three possible groups at its chain ends: nitrile, guanidine or amine. Oligomers of PHMB (Fig. 1) generally available from commercial sources may be used to fabricate the dressing, where n=6 to100, preferably 6-20. Mixtures of oligomers, in commercial PHMB have three possible groups at the polymer chain ends: nitrile, guanidine or amine, and mixtures of these can be used as may be purchased from commercial sources according to the present invention. Suitable salts of PHMB include, but are not limited to salts of hydrochlorides, hydrobromides, gluconates, acetates, ascorbates and citrates.

The chain length (Figure 1, n) of PHMB may have a maximum size of 40-42 subunits. There exists an equilibrium of polymerization/depolymerization, with depolymerization occurring by biguanide break resulting in one cyanamide chain-end and one guanidine chain-end. Longer chains (Figure 1, n>40 subunits) will break up into smaller chains that can undergo interchange reactions, complicating the estimation of the true molecular weight distribution. This process can explain the maximum chain length around n= 40 subunits with a weight-averaged degree of polymerization of n=12 [cited in G. F. de Paula, Physical and Chemical Characterization of Poly(hexamethylene biguanide) Hydrochloride Polymers 2011, 3, 928-941].

PHMB is soluble in water, alcohols and glycols, and is incompatible with anionic surfactants and soaps; it is not soluble in oils and hydrocarbons and silicone elastomers. It should be kept at a pH below 8.0, and should not be heated above 80°C. It is however, stable in the presence of light, pH 4-10, and up to 80°C. In the silicone adhesive of this invention PHMB is stable to the curing temperature of 140°C, and to autoclaving at 121°C. PHMB is a heterogeneous mixture of polymers. The basic molecular chain of PHMB can be repeated from two to about forty times with increasing polymer chain length correlating with increasing antiseptic/antimicrobial efficacy. While the effect of PHMB on managing bioburden is well-known, exposure of viral particles to PHMB causes them to clump together as aggregates. Comparative tests of PHMB's biocompatibility (measurement of an antiseptic/antimicrobial agent's activity in relation to its cytotoxicity) against other commonly used standard of care therapies have demonstrated its superiority over chlorhexidine, povidone-iodine, triclosan, silver and sulfadiazine.

In aspects of the invention to the silicone is also added a surfactant (surface active agent) which reduces the surface tension of a fluid allowing it to better wet a surface. Any amphoteric surfactant having this property can be utilized in the silicone adhesive of the invention such as betaine and glycerin. The amphoteric surfactant is present in an amount of up to about 2%. In aspects about 0.1% to about 2% v.v. In aspects up to about 1%, up to about 1.5%, and up to about 2%. In aspects the silicone adhesive comprises glycerin, in aspects about 2.0% glycerin is utilized.

The amount of antimicrobial for use in the present invention, in aspects PHMB, can range from about 0.1 to about 1%, or from about 0.3 to about 0.5% or about 0.3% The amount of chelator for use in the dressing of the invention, such as the EDTA, can range from about 0 to about 1%, or from about 0.1 to about 0.5% or about 0.01%. One of skill in the art can determine the effective amounts with the teachings of these ranges. By effective is meant that the dressings of the invention can serve their purpose for wound care management and are substantially antimicrobial.

In an aspect of the invention, the antimicrobial silicone adhesive dressing is made by loading each of PHMB and EDTA in the mixture and glycerin prior to curing.

The silicone adhesive is formulated from two parts. Part A is the primary silicone polymer with catalysts, and Part B contains the crosslinking agent. The silicone is a two part platinum cure silicone adhesive product supplied from the Dow Corning Corporation. The antimicrobial chemicals (PHMB, and EDTA) and humectant (glycerin) are added to the silicone through an addition to the Part B component. In an aspect, the mixing ratio of Part A:Part B is 0.99:1 but may vary. The ingredients are added to Part A component in a container and mixed uniformly. Once the silicone is mixed thoroughly, it is coated onto a polyurethane backing that is fed through a convection oven. As the polyurethane backing is pulled through the oven, the silicone mixture is coated to a specific thickness. The oven subjects the silicone mixture to a specified temperature for a given length of time. The elevated temperature aids in the cure of the silicone coating. Oven temperatures for curing may be from about 130°C to about 150°C, in aspects about 140°C. As the coated substrate leaves the oven a polycarbonate liner is laminated to the cured silicone surface.

The thickness of the silicone coating is about 0.1 mm to about 0.5 mm, in aspects about 0.18 mm. The dressing range can be from about 0.01mm to about 5 mm, preferably for thin film SSA application it can range from about 0.01 to about 1mm, and in aspects from about 0.1 to about 0.5 mm, and in further aspects from about 0.14 to about 0.2 mm, and in aspects about 0.18 mm thick; the polyurethane backing layer is up to about 28 microns thick One of skill in the art would understand that the backing layer thickness may be dependent on the particular application and thus can vary and in aspects be thicker than 28 microns.

The silicone adhesive dressings of the invention can be fabricated onto the backing material and an optional liner is applied to the tissue contact surface for use prior to packaging such that the dressing does not adhere to the packaging material per se. Thus a dressing for clinical use may be made with a backing layer that is substantially air permeable and substantially permeable to moisture vapour, yet substantially impermeable to liquids, microorganisms and viruses; having a silicone antimicrobial thin film thereon as described herein; and a liner such as a polycarbonate or similar type liner to protect the tacky surface as packaged. The dressings can be packaged as kits for wound care/would management use.

The invention will be further illustrated by reference to the following non-limiting Examples. All parts and percentages are expressed as parts by weight unless otherwise indicated.

### Examples: these are non-limiting and encompass embodiments of the invention.

### Example 1: Formation of Silicone Gel Adhesive Dressing

A mixture was formed of; (1) a liquid containing silicone elastomer composition, in which the adhesive elastomers are based on a platinum-catalyzed polydimethyl-siloxane composition that cures at a variety of temperatures from ambient to 140°C, without the formation of by-products, and (2) a PHMB compound plus the metal chelator, EDTA, in the hydrophobic silicone mixture. Glycerol was added in some of the mixtures. The mixture was allowed to form a transparent adhesive material under appropriate and suitable conditions of temperature and pressure in the presence or absence of the catalyst, such as platinum. The gel material was applied to a polyurethane backing layer.

The cured silicone tacky gel adhesive contact layer contained 0.01% tetramethylvinylcyclosilioxane, had a total thickness of about 0.18 mm on a polyurethane backing carrier of about 28 micron (0.028 mm) thickness. The thickness of the contact layer may be modified to be more or less than 0.18 mm in thickness as may be required.

A cured silicone tacky gel adhesive contact layer containing about 0.01% tetramethylvinylcyclosilioxane, having a total thickness of about 0.18 mm on a polyurethane backing carrier was also formed without catalyst. The thickness of the contact layer may be modified to be more or less than 0.18 mm in thickness as may be required.

### Methodology

a. Weighed out 249 grams of Part B silicone (Dow Corning: DC7-9900) for compounding a 500 gram total weight sample size of the mixture.
b. Weighed out 0.05 gm EDTA and 1.5 gm PHMB.
c. Added 2% v/v glycerin
d. Added b) and c) chemicals one at a time to the silicone under constant mixing and after thorough mixing, vacuum mix mixture.
e. Mixed the material of step d) above with 249 gm of Part A silicone.
f. Coated the material onto a polyurethane sheet to a thickness of up to about 28 microns in at an oven temperature of 140°C at a dwell time of two minutes.
g. Loading of each of PHMB and EDTA in the cured final dressing.
   PHMB amount in final coating = 0.3% wgt
   EDTA amount in final coating = 0.01% wgt
   Glycerin amount in final coating = 2% v/v
   Silicone gel = 97.69%
   Total = 100%

The composition had excellent and instantaneous tack, atraumatic removal from skin, is hypoallergenic, had the ability to be removed and repositioned, and is transparent. Curing times, temperature and pressures for forming the silicone adhesive composition containing the PHMB and EDTA are known to one of skill in the art. For example, cohesive and self-adhering silicone materials may be produced from platinum catalyzed polydimethyl-siloxane that will cure at a variety of temperatures from ambient to 140°C.

A single-coated wound dressing is made with adhesive side exposed on only one side of polyurethane backing layer. Backings can be made of cloth, various films, foams, and a range of other materials. Single-coated products may be manufactured either with or without a release liner as is well-known in the art. Silicone adhesion and release test methods utilized ASTM D3330. The silicone coat weight test procedure was developed using ASTM D899-00.

### Example 2: Formation of Silicone Gel Adhesive Dressing

To Part B as described above was added the following:
- 2% glycerin;
- 0.3% PHMB;
- 0.01% EDTA.

Part A as described above was then added to Part B mixture and mixed as described above. The composition had excellent and instantaneous tack, atraumatic removal from skin, is hypoallergenic, had the ability to be removed and repositioned, and is transparent.

### Antimicrobial Assay

Test samples of the antimicrobial silicone adhesive with PHMB and EDTA were cut into 5 cm x 5 cm pieces and placed with the adhesive side facing up on LB agar microbiology plates; control silicone adhesive samples had no PHMB or EDTA. A microliter droplet of microorganism adjusted to 105 CFU/mL of either *Staphylococcus aureus* or *Candida albicans* was placed directly onto the adhesive surface in 4 replicate locations. The plates with adhesive pieces and microorganisms were incubated at 37°C for 18 hours. After 18 hours the droplets were taken up with a pipette and transferred directly onto the surface of new LB plates and incubated for 18 hours at 37°C. Growth of microorganism was assessed visually. No growth of either microorganism was indicative of antimicrobial activity.

Liquid samples on PHMB and EDTA-containing silicone adhesive with microbes taken up after 18 hours and transferred to new LB plates showed no growth after incubation for 18 hours, indicating antimicrobial activity of the silicone adhesive. Antimicrobial activity was assessed for longer periods and it was found to provide microbiocidal activity for at least up to 7 days.

In understanding the scope of the present application, the articles "a", "an", "the", and "said" are intended to mean that there are one or more of the elements. Additionally, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms "including", "having" and their derivatives.

It will be understood that any aspects described as "comprising" certain components may also "consist of" or "consist essentially of," wherein "consisting of" has a closed-ended or restrictive meaning and "consisting essentially of" means including the components specified but excluding other components except for materials present as impurities, unavoidable materials present as a result of processes used to provide the components, and components added for a purpose other than achieving the technical effect(s) described herein. For example, a composition defined using the phrase "consisting essentially of" encompasses any known pharmaceutically acceptable additive, excipient, diluent, carrier, and the like. Typically, a composition consisting essentially of a set of components will comprise less than 5% by weight, typically less than 3% by weight, more typically less than 1% by weight of non-specified components.

It will be understood that any component defined herein as being included may be explicitly excluded from the claimed invention by way of proviso or negative limitation. For example, in aspects, certain of the recited components if desired can be explicitly excluded from the compositions and methods described herein.

In addition, all ranges given herein include the end of the ranges and also any intermediate range points, whether explicitly stated or not.

Finally, terms of degree such as "substantially", "about" and "approximately" as used herein mean a reasonable amount of deviation of the modified term such that the end result is not significantly changed. These terms of degree should be construed as including a deviation of at least ±5% of the modified term if this deviation would not negate the meaning of the word it modifies.

## Claims

1. A method for making an antimicrobial adhesive silicone for an adhesive dressing, the method comprising mixing together;
a liquid containing silicone with a silicone dispersion of polyhexamethylene biguanide hydrochloride (PHMB) and EDTA that are not soluble in said liquid and remain as particulates, and optional humectant;
and allowing the mixture to cure under suitable temperature and pressure.

2. The method of claim 1, wherein said cured antimicrobial adhesive silicone comprises 0.1 to 1%, or from 0.3 to 0.5% or 0.3% of PHMB.

3. The method of claim 1 or 2, wherein said cured antimicrobial adhesive silicone comprises up to 0.1% EDTA, or from 0.01 to 1%, or from 0.1 to 0.5% EDTA.

4. The method of any one of claims 1 to 3, wherein said humectant is present in an amount of up to about 2% in said cured antimicrobial adhesive silicone.

5. The method of claim 4, wherein said humectant is glycerin.

6. The method of any one of claims 1 to 5, wherein the curing is effected in the presence of a platinum catalyst.

7. The method of any one of claims 1 to 6, further comprising adding a pharmaceutical agent to said dispersion.

8. The method of any one of claims 1 to 7, wherein said mixture is coated onto a backing material prior to curing and thus forming a thin sheet, said backing material is selected from the group consisting of polyurethane, polyester, vinyl, cellulose, oxycellulose, rayon, viscose and composite biomaterials.

9. The method of any one of claims 1 to 8, wherein a liner is laminated to said cured antimicrobial adhesive silicone on a tissue and/or skin contact surface.

10. An adhesive antimicrobial dressing comprising:
a transparent and adhesive silicone sheet or film cured from a liquid containing silicone, the sheet comprising humectant and dispersed particulates of polyhexamethylene biguanide hydrochloride (PHMB) and EDTA that are not soluble in said liquid containing silicone;
a backing layer on one side of said sheet; and
an optional liner on a tissue and/or skin contact side.

11. The dressing of claim 10, wherein the amount of PHMB in said sheet is up to about 5% by weight of said dressing and wherein the amount of EDTA in said sheet is 0.1% EDTA, or from 0.01 to 1%, or from 0.1% to 0.5%.

12. The dressing of claim 10 or 11, wherein said humectant is glycerin provided in an amount of up to 2% v/v.

13. The dressing of any one of claims 10 to 12, wherein said dressing has a thickness of up to about 5 mm and said dressing is one or more of self-adhering, flexible, transparent, re-positionable and washable.

14. The dressing of any one of claims 10 to 13, wherein said backing layer is made of a material selected from the group consisting of polyurethane, polyester, vinyl, cellulose, oxycellulose, rayon, viscose and composite biomaterials.

15. The antimicrobial adhesive silicone dressing of any one of claims 10 to 14 for use in a method for treating wound areas and surgical incisions or for preventing or minimizing infection of a wound or incision site or needle puncture, the method comprising contacting the wound, the surgical incision or the needle puncture with the antimicrobial adhesive silicone dressing.

## Patentansprüche

1. Verfahren zur Herstellung eines antimikrobiellen Klebesilikons für einen Klebeverband, wobei das Verfahren das Zusammenmischen umfasst von
einer Flüssigkeit, die Silikon mit einer Silikon-Dispersion von Polyhexamethylenbiguanidhydrochlorid (PHMB) und EDTA enthält, die in der Flüssigkeit nicht löslich sind und als Partikel bestehen bleiben, und wahlweise eines Feuchthaltemittels;
und das Aushärten des Gemischs bei geeigneter Temperatur und Druck.

2. Verfahren nach Anspruch 1, wobei das gehärtete antimikrobielle Klebesilikon 0,1 bis 1% oder 0,3 bis 0,5% oder 0,3% PHMB umfasst.

3. Verfahren nach Anspruch 1 oder 2, wobei das gehärtete antimikrobielle Klebesilikon bis zu 0,1% EDTA oder von 0,01 bis 1% oder von 0,1 bis 0,5% EDTA umfasst.

4. Verfahren nach einem der Ansprüche 1 bis 3, wobei das Feuchthaltemittel in dem gehärteten antimikrobiellen Klebesilikon in einer Menge von bis zu etwa 2% vorliegt.

5. Verfahren nach Anspruch 4, wobei das Feuchthaltemittel Glycerin ist.

6. Verfahren nach einem der Ansprüche 1 bis 5, wobei das Härten in Gegenwart eines Platinkatalysators bewirkt wird.

7. Verfahren nach einem der Ansprüche 1 bis 6, das ferner das Zugeben eines pharmazeutischen Mittels zu der Dispersion umfasst.

8. Verfahren nach einem der Ansprüche 1 bis 7, wobei das Gemisch vor dem Härten auf ein Trägermaterial aufgetragen wird und so ein dünnes Blatt gebildet wird, wobei das Trägermaterial ausgewählt ist aus der Gruppe bestehend aus Polyurethan, Polyester, Vinyl, Cellulose, Oxycellulose, Rayon, Viskose und Verbundbiomaterialien.

9. Verfahren nach einem der Ansprüche 1 bis 8, wobei eine Schutzfolie auf das gehärtete antimikrobielle Klebesilikon auf einer Gewebe- und/oder Hautkontaktoberfläche laminiert wird.

10. Antimikrobieller Klebeverband, der Folgendes umfasst:
ein transparentes und klebendes Silikonblatt oder -folie, die aus einer Silikon enthaltenden Flüssigkeit gehärtet sind, wobei das Blatt Feuchthaltemittel und dispergierte Partikel von Polyhexamethylenbiguanidhydrochlorid (PHMB) und EDTA umfasst, die in der silikonhaltigen Flüssigkeit nicht löslich sind;
eine Trägerschicht auf einer Seite des Blattes; und
eine wahlweise Schutzfolie auf einer Gewebe- und/oder Hautkontaktseite.

11. Verband nach Anspruch 10, wobei die Menge an PHMB in dem Blatt bis zu etwa 5 Gew.-% des Verbandes beträgt und wobei die Menge an EDTA in dem Blatt 0,1% EDTA oder von 0,01 bis 1% oder von 0,1% bis 0,5% beträgt.

12. Verband nach Anspruch 10 oder 11, wobei das Feuchthaltemittel Glycerin ist, das in einer Menge von bis zu 2 Vol.-% vorgesehen wird.

13. Verband nach einem der Ansprüche 10 bis 12, wobei der Verband eine Dicke von bis zu etwa 5 mm aufweist und der Verband selbstklebend, flexibel, transparent, wiederpositionierbar und/oder waschbar ist.

14. Verband nach einem der Ansprüche 10 bis 13, wobei die Trägerschicht aus einem Material hergestellt ist, das ausgewählt ist aus der Gruppe bestehend aus Polyurethan, Polyester, Vinyl, Cellulose, Oxycellulose, Rayon, Viskose und Verbundbiomaterialien.

15. Antimikrobieller Klebesilikonverband nach einem der Ansprüche 10 bis 14 zur Verwendung in einem Verfahren zur Behandlung von Wundbereichen und chirurgischen Einschnitten oder zur Verhinderung oder Minimierung einer Infektion einer Wunde oder einer Einschnittstelle oder einer Nadelpunktion, wobei das Verfahren das in Kontakt bringen der Wunde, des chirurgischen Einschnitts oder der Nadelpunktion mit dem antimikrobiellen Klebesilikonverband umfasst.

## Revendications

1. Procédé de fabrication d'une silicone adhésive antimicrobienne pour un pansement adhésif, le procédé comprenant le mélange ensemble ;
d'un liquide contenant de la silicone avec une dispersion dans la silicone de chlorhydrate de polyhexaméthylène biguanide (PHMB) et d'EDTA qui ne sont pas solubles dans ledit liquide et restent sous la forme de particules, et un humectant facultatif ;
et le fait de laisser le mélange durcir à une température et une pression adaptées.

2. Procédé selon la revendication 1, dans lequel ladite silicone adhésive antimicrobienne durcie comprend de 0,1 à 1 %, ou de 0,3 à 0,5 % ou 0,3 % de PHMB.

3. Procédé selon la revendication 1 ou 2, dans lequel ladite silicone adhésive antimicrobienne durcie comprend au plus 0,1 % d'EDTA, ou de 0,01 à 1 %, ou de 0,1 à 0,5 % d'EDTA.

4. Procédé selon l'une quelconque des revendications 1 à 3, dans lequel ledit humectant est présent en une quantité d'au plus environ 2 % dans ladite silicone adhésive antimicrobienne durcie.

5. Procédé selon la revendication 4, dans lequel ledit humectant est la glycérine.

6. Procédé selon l'une quelconque des revendications 1 à 5, dans lequel le durcissement est effectué en présence d'un catalyseur de platine.

7. Procédé selon l'une quelconque des revendications 1 à 6, comprenant en outre l'ajout d'un agent pharmaceutique à ladite dispersion.

8. Procédé selon l'une quelconque des revendications 1 à 7, dans lequel ledit mélange est revêtu sur un matériau de support avant le durcissement formant ainsi une feuille mince, ledit matériau de support est sélectionné dans le groupe constitué de polyuréthane, polyester, vinyle, cellulose, oxycellulose, rayonne, viscose et biomatériaux composites.

9. Procédé selon l'une quelconque des revendications 1 à 8, dans lequel un revêtement est stratifié sur ladite silicone adhésive antimicrobienne durcie sur une surface de contact avec un tissu et/ou avec la peau.

10. Pansement antimicrobien adhésif comprenant :
une feuille ou film de silicone adhésive et transparente durci à partir d'un liquide contenant de la silicone, la feuille comprenant un humectant et des particules dispersées de chlorhydrate de polyhexaméthylène biguanide (PHMB) et d'EDTA qui ne sont pas solubles dans ledit liquide contenant de la silicone ;
une couche de support sur le côté de ladite feuille ; et
un revêtement facultatif sur un côté de contact avec le tissu et/ou avec la peau.

11. Pansement selon la revendication 10, dans lequel la quantité de PHMB dans ladite feuille est d'au plus environ 5 % en poids dudit pansement et dans lequel la quantité d'EDTA dans ladite feuille est de 0,1 % d'EDTA, ou de 0,01 à 1 %, ou de 0,1 % à 0,5 %.

12. Pansement selon la revendication 10 ou 11, dans lequel ledit humectant est la glycérine fournie en une quantité d'au plus 2 % v/v.

13. Pansement selon l'une quelconque des revendications 10 à 12, dans lequel ledit pansement a une épaisseur d'au plus environ 5 mm et ledit pansement est un ou plusieurs d'autocollant, souple, transparent, repositionnable et lavable.

14. Pansement selon l'une quelconque des revendications 10 à 13, dans lequel ladite couche de support est constituée d'un matériau sélectionné dans le groupe constitué du polyuréthane, du polyester, du vinyle, de la cellulose, de l'oxycellulose, de la rayonne, de la viscose et de biomatériaux composites.

15. Pansement de silicone adhésive antimicrobienne selon l'une quelconque des revendications 10 à 14, pour son utilisation dans un procédé de traitement de zones de plaie et d'incisions chirurgicales ou de prévention ou de minimisation de l'infection d'une plaie ou d'un site d'incision ou d'une piqûre d'aiguille, le procédé comprenant la mise en contact de la plaie, de l'incision chirurgicale ou de la piqûre d'aiguille avec le pansement de silicone adhésive antimicrobienne.
